Europäisches Patentamt

European Patent Office

Office européen des brevets

(11) Publication number: **0 408 160 A1**

# EUROPEAN PATENT APPLICATION

(21) Application number: 90250173.3

(22) Date of filing: 04.07.90

(51) Int. Cl.5: **A61B 17/36**

(30) Priority: **10.07.89 JP 177443/89**

(43) Date of publication of application:
**16.01.91 Bulletin 91/03**

(84) Designated Contracting States:
**DE FR SE**

(71) Applicant: **Kabushiki Kaisha TOPCON**
**75-1, Hasunuma-cho Itabashi-ku**
**Tokyo(JP)**

(72) Inventor: **Koizumi, Hiroshi, c/o Kabushiki**
**Kaisha Topcon**
**75-1, Hasunuma-cho, Itabashi-ku**
**Tokyo(JP)**
Inventor: **Kijima, Masatsugu, c/o Kabushiki**
**Kaisha Topcon**
**75-1, Hasunuma-cho, Itabashi-ku**
**Tokyo(JP)**
Inventor: **Tomioka, Yuko, c/o Kabushiki**
**Kaisha Topcon**
**75-1, Hasunuma-cho, Itabashi-ku**
**Tokyo(JP)**

(74) Representative: **Pfenning, Meinig & Partnerner**
**Kurfürstendamm 170**
**D-1000 Berlin 15(DE)**

(54) Laser surgical apparatus.

(57) A laser surgical apparatus having in a housing
(1) a laser source, an inserting hole (7) for a hand-
piece (8), and an output power display means (4) for
displaying an output power value of a laser beam
emitted from the tip of a handpiece.

The housing also has auto-changing means (11)
for changing automatically the output power value
corresponding to the handpiece connected to the
housing to another output power value corresponding to the different type of handpiece connected to
the housing in the time when a different type of
handpiece is connected to the housing.

In other words, when a type of handpiece is
connected to the housing, the auto-changing means
distinguish the type, and thereby the output power
display means displays an output power value of the
laser beam corresponding to the type of handpiece.

FIG. 2

# LASER SURGICAL APPARATUS

## BACKGROUND OF THE INVENTION

### FIELD OF THE INVENTION

This invention relates to an improvement of a laser surgical apparatus having in a housing a laser source, and also on the frontage of the housing an output power display means and an inserting hole for a handpiece from which a laser beam is emitted.

### DESCRIPTION OF THE PRIOR ART

Heretofore, there is known a laser surgical apparatus having a laser source in a housing. The laser surgical apparatus also has an output power display means and an inserting hole for a handpiece from which a laser beam is emitted. Said output power display means and said inserting hole are on the frontage of the housing.

After said handpiece is inserted into the hole, said output power display means performs a function to display an output power value of the laser beam emitted from the tip of said handpiece. Thus an operator can perform a surgical operation safely.

However, the operator may, for some reason or other, use a different type of handpiece which should not be originally used for the apparatus. If the different type of handpiece is inserted to the hole and the laser beam is emitted from the tip of the handpiece, the displayed output value will mismatch the output power of the laser beam emitted from the handpiece. That is, the laser beam output value from the handpiece does not accurately correspond to that of the output power display means mounted in the housing.

And accordingly, overconfidence in the displayed value will cause much danger to the surgical operation because the output power of the emitted laser beam is occasionally too strong.

## SUMMARY OF THE INVENTION

To perform a surgical operation safely without the foregoing danger, the laser surgical apparatus of this invention is constituted as follows;

A laser source and an inserting hole for a handpiece are mounted in an apparatus housing. Preferably, the inserting hole should be mounted on the frontage of the housing. And output power display means for displaying an output power value of a laser beam emitted from the tip of the handpiece also should be mounted on the frontage.

When a different type of handpiece is connected to the housing, auto-changing means for displaying will change automatically said output power value corresponding to said handpiece connected to the housing to another output power value corresponding to the different type.

In other words, a handpiece connection to the housing makes the auto-changing means distinguish the type of handpiece, and thereby the output power display means displays the output power value of a laser beam corresponding to the type of handpiece.

## BRIEF DESCRIPTION OF THE DRAWINGS

Figure 1 and Figure 2 are partially sectional views of auto-changing means of the laser surgical apparatus according to the invention;

Figure 3 is a perspective view showing a whole structure of the laser surgical apparatus;

Figure 4 is a partially sectional view of another embodiment of the laser surgical apparatus;

Figure 5 is a partially sectional view of still another embodiment of the laser surgical apparatus;

Figure 6 and Figure 7 are partially sectional views showing other configurations to solve problems in accordance with the present invention.

## DESCRIPTION OF THE PREFERRED EMBODIMENT

The embodiments of the present invention will be described hereinafter with reference to the accompanying drawings.

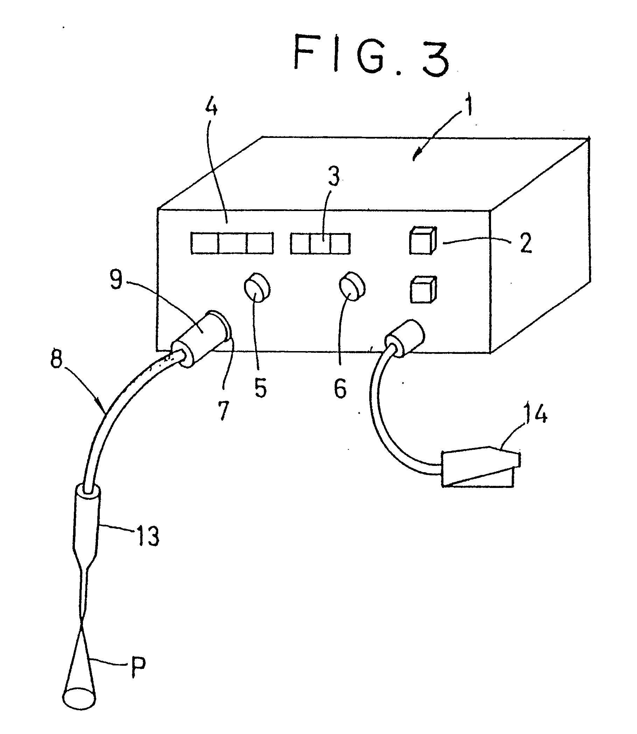

Figure 3 is a perspective view showing a whole structure of the laser surgical apparatus according to the present invention. In Fig.3 ,the numeral 1 designates the housing of the apparatus. The frontage of the housing 1 mounts a power switch 2, a radiation time display portion 3, an output power display portion 4, a control knob 5 for output power, a control knob 6 for radiation time, and an inserting hole 7 for a handpiece. The electrical source in the housing is turned on or off by the power switch 2. The radiation time is set by the control knob 6. The output power value of the laser beam emitted from a handpiece 8 is set by the control knob 5. And the radiation time display portion 3 displays a radiation time set by the control knob 6. The output power display portion 4 displays an output power value set by the control knob 5.

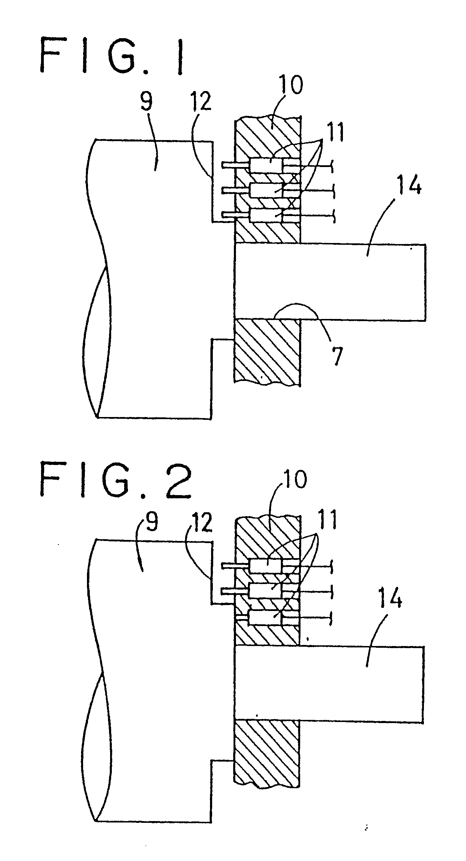

The handpiece 8 comprises an inserted portion 9 inserted into the hole 7 and a handle 13. Referring to Figs.1 and 2, a plurality of mechanical switches 11 as an auto-changing means are mounted in the wall 10 of the hole 7. The inserted portion 9 has lack portions 12 mounted at the end portion of the portion 9 corresponding to the type of handpiece 8. Fig.1 shows that three switches 11 are in the off state by the lack portion. Referring to Fig.2, one switch is on and the other two are off by the lack portion.

Each mechanical switch 11 is connected to a control unit (not shown). The control unit discriminates the type of handpiece connected to the housing 1 by the on or off signals from the mechanical switches 11. That is , the mechanical switches 11 cooperate with the control unit in discriminating the type of handpiece connected to the housing 1.

The control unit can also control the output power of the laser beam P emitted from the tip of the handle 13, so as to emit the laser beam P of the output power value set by the knob 5 . The numeral 14 is a foot-type radiation switch for emitting the laser beam by pushing with a foot.

An operator may, for some reason or other, use another type of handpiece which is not standard in the core diameter of the fiber or in numerical appertures, for example. When the different type of handpiece is inserted into the hole 7, the mechanical switches 11 ,together with the control unit, distinguish the type of handpiece, and thereby the output power display means 4 displays the output power value of a laser beam corresponding to the type of handpiece.

If the output power value is set "90" , for example, by the knob 5 , the output power display means 4 Hill display an output power value "90" in the standard type of handpiece. But in a different type of handpiece, the output power display means will display the value "85", for example, even though the output power value is set "90" by the knob 5. That value "85" is the correct one for the different type. The "85" is again changeable to "90" by the knob 5 if the operator wants to set the emitted output-power at "90". In the conventional apparatus , however, the displayed value shows an unchanged "90" when the different type of handpiece is inserted. And moreover, the emitted-output power value is in actual fact "85".

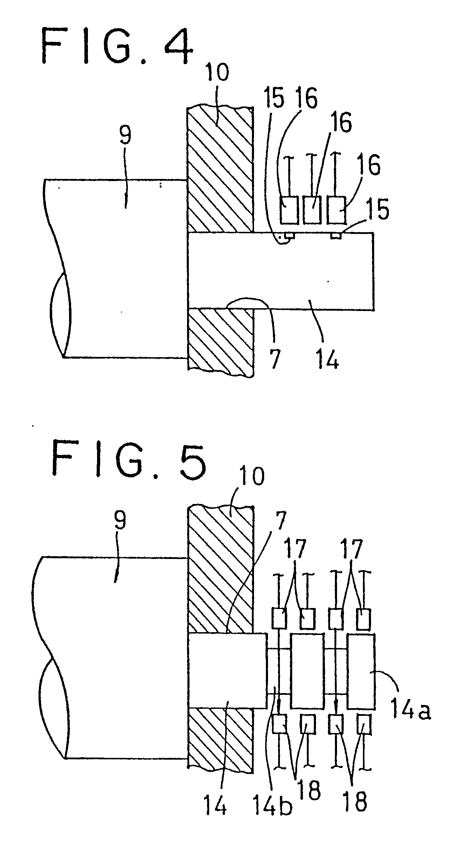

In Fig.4 showing a second embodiment of the invention, one or more magnets 15 are mounted in the outerface portion of the optical connector barrel 14 of the inserted portion 9. The configuration of the magnets 15 is determined corresponding to the type of handpiece 8. The magent switches mounted in the vicinity of the hole 7 are faced to the optical connector barrel 14 so as to distinguish the type of said handpiece.

Fig.5 shows a third embodiment of the laser surgical apparatus of the present invention. The optical connector barrel 14 of the inserted portion 9 is comprised of large diameter portions 14a and small diameter portions 14b corresponding to the type of the handpiece 8. The auto-changing means is comprised of the light-switching means having a plurality of light-emitting elements 17 and light-receiving elements 18. Each of light-emitting elements 17 is opposite to each of light-receiving elements 18 so as to distinguish the type of said handpiece.

In accordance with the third embodiment of the invention, the type of handpiece is discriminated by detecting the combinations of the large diameter portions and the small diameter portions with the light switches.

Besides the above means, a bar code means is to be used in order to distinguish the type of handpiece 8.

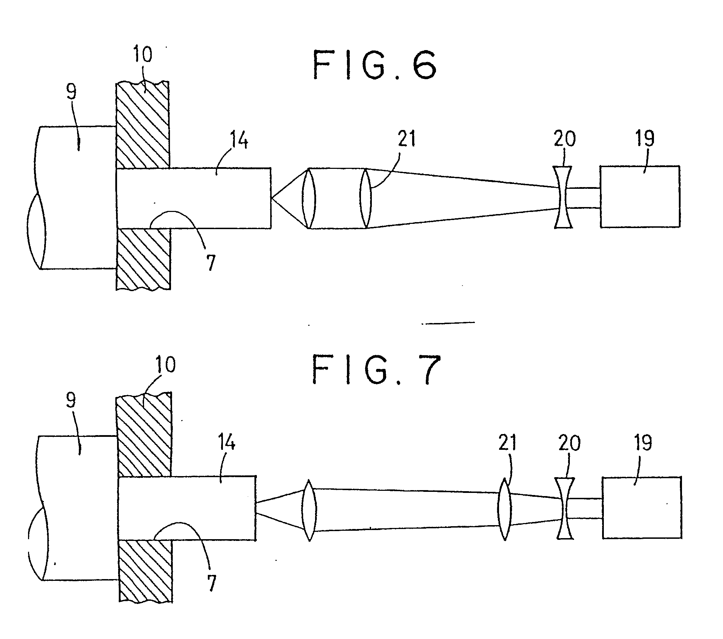

Figures 6 and 7 are other configurations for resolving problems according to the invention. Both a concave lens 20 and a convex lens 21 comprise the beam expander lens of the laser beam 19. The displayed value of the output display means 4 accords with that of the output power of the laser beam P emitted from the tip of the handle 13 by moving the convex lens 21 toward the axis in the time when the different type of the handpiece 8 is inserted into the hole 7.

The configurations shown in Figs.6 and 7 have the same effect as the foregoing auto-changing means. Additionally, a similar effect is gained by moving the concave lens 20 toward the axis in the time when the different type of the handpiece 8 is inserted into the hole 7.

It is also possible to adopt an arrangement that a convergent lens is mounted in a turret, and then the control unit rotates the turret in order that the handpiece 8 corresponds to the convergent lens inserted into the optical path of the laser beam.

## Claims

1. A laser surgical apparatus having a laser source in a housing, an inserting hole for a handpiece, and an output power display means for displaying an output power value of a laser beam emitted from the tip of a handpiece,
characterized by said housing having an auto-changing means for changing automatically said output power value corresponding to said handpiece connected to the housing to another output power value corresponding to the different type of handpiece connected to the housing in the time when a different type of handpiece is connected to the housing.

2. A laser surgical apparatus according to claim 1, wherein said auto-changing means is mounted in the vicinity around said hole.

3. A laser surgical apparatus according to claim 2, wherein said auto-changing means comprises a plurality of mechanical switches mounted in the wall around said hole, so as to distinguish the type of said handpiece.

4. A laser surgical apparatus according to claim 2, wherein said handpiece comprises a handle and an inserted portion;
said inserted portion having a connector barrel;
said auto-changing means comprising a plurality of magnet switches and one or more magnets mounted in the the connector barrel, so as to distinguish the type of said handpiece.

5. A laser surgical apparatus according to claim 2, wherein said handpiece comprises a handle and an inserted portion;
said inserted portion having a connector barrel;
said connector barrel having large diameter portions and small diameter portions corresponding to each type of handpiece;
said auto-changing means comprising a plurality of light-switch means;
each light switch means comprising a light-emitting element and a light-receiving element;
said light-emitting element opposite to said light-receiving element through said connector barrel, so as to distinguish the type of said handpiece.

6. A laser surgical apparatus according to claim 1, wherein said auto-changing means comprises bar code means.

7. A laser surgical apparatus having a laser source in a housing, and also having on the frontage of the housing an inserting hole for a handpiece, and an output power display means for displaying a output power value of a laser beam emitted from the tip of said handpiece,
characterized by said housing having an auto-changing means for changing automatically said output power value corresponding to said handpiece connected to the housing to another output power value corresponding to the different type of handpiece connected to the housing in the time when the different type of handpiece is connected to the housing.

8. A laser surgical apparatus according to claim 7;
wherein said handpiece comprises a handle and an inserted portion;
said inserted portion having lack portions for distinguishing the type of said handpiece;
said auto-changing means comprising a plurality of mechanical switches;
said mechanical switches being turned on or off by said lack portions.

# FIG. 1

# FIG. 2

# FIG. 3

FIG.4

FIG.5

# FIG.6

# FIG.7

**European Patent Office**

# EUROPEAN SEARCH REPORT

Application Number

**EP 90 25 0173**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | GB-A-2 210 706 (PILKINGTON MEDICAL SYSTEMS LTD) <br> * Page 8, line 26 - page 9, line 24; figure 7 * <br> − − − | 1-8 | A 61 B 17/36 |
| Y | EP-A-0 300 317 (SIEMENS) <br> * Column 6, line 34 - column 7, line 46; figures 1-3 * <br> − − − − − | 1-8 | |

**TECHNICAL FIELDS SEARCHED (Int. Cl.5)**

A 61 B
A 61 C
G 05 B

The present search report has been drawn up for all claims

| Place of search | Date of completion of search | Examiner |
|---|---|---|
| The Hague | 16 October 90 | MOERS R.J. |

CATEGORY OF CITED DOCUMENTS

X : particularly relevant if taken alone
Y : particularly relevant if combined with another
    document of the same catagory
A : technological background
O : non-written disclosure
P : intermediate document
T : theory or principle underlying the invention

E : earlier patent document, but published on, or after
    the filing date
D : document cited in the application
L : document cited for other reasons

& : member of the same patent family, corresponding
    document